# FASCICULE DE BREVET EUROPEEN

(11) **EP 0 973 466 B1**
(45) Date de publication et mention de la délivrance du brevet: **09.04.2003**
(21) Numéro de dépôt: 98901922.9
(22) Date de dépôt: 18.02.1998
(51) Int. Cl.: A61F 11/00, A47L 17/00, A61F 13/38

(54) **CURE-OREILLES**
GEHÖRGANGREINIGER
EAR-SPOON

(30) Priorité: 24.03.1997 CH 70797
(43) Date de publication de la demande: 26.01.2000
(73) Titulaire: Arsline S.A., 6833 Vacallo (CH)
(72) Inventeur: TURRI, Achille, CH-6834 Morbio Inferiore (CH)
(74) Mandataire: AMMANN PATENTANWAELTE AG BERN
(86) Numéro de dépôt international: CH9800063
(87) Numéro de publication internationale: WO98042284

(56) Documents cités:
- EP-A- 0 158 543
- EP-A- 0 556 432
- FR-A- 1 582 734
- US-A- 1 693 581
- US-A- 1 980 826

## Description

La présente invention a pour objet un ustensile ou instrument de nettoyage ou de traitement à usages et applications multiples, notamment d'hygiène corporelle, répondant à la définition selon le préambule de la revendication 1.

On connaît différents produits de ce genre, en particulier, lorsqu'il s'agit d'hygiène corporelle, par exemple pour le nettoyage du conduit auditif externe, le coton-tige, lequel est constitué d'une tige mince dont au moins l'une des deux extrémités est pourvue d'un élément nettoyant, en général du coton hydrophile ou de l'ouate. Si les cotons-tiges sont de fabrication simple et peu onéreuse, leur pouvoir nettoyant, en revanche, est assez médiocre et limité. En effet, la tête nettoyante étant mince (son diamètre ne dépasse que légèrement celui de la tige), l'utilisateur doit effectuer avec sa main tenant la tige un mouvement de rotation de sorte à appliquer la tête nettoyante successivement contre toutes les génératrices ou endroits du conduit à nettoyer ou à traiter. La dégradation de la tête nettoyante qu'entraîne ce mouvement de rotation s'accélère rapidement lorsque l'utilisateur imprime à la tige un deuxième mouvement de rotation se superposant au premier, consistant en une rotation simultanée de la tige autour de son axe pour améliorer l'effet nettoyant. Ensuite, la mise en oeuvre du coton-tige est tout autre qu'idéale, voire inefficace, lorsqu'un liquide est auparavant introduit dans le conduit ou lorsque la tête est, avant utilisation, trempée dans un produit pharmaceutique, cosmétique ou détergent par exemple (qu'on appellera plus loin uniformément fluide actif).
Enfin, compte tenu du diamètre des cotons-tiges, leur mise en oeuvre n'est pas sans risque de dommages sur le tympan.

Aussi dans une forme élaborée, les têtes de certains cotons-tiges présentent à leur base un bourrelet limitant la distance de leur introduction dans le conduit, ce qui amoindrit ce risque (sans pourtant l'éliminer totalement), mais laisse subsister les autres inconvénients décrits.
C'est pourquoi d'autres dispositifs encore ont été proposés en vue de les pallier.

US-1,693,581 est un instrument de nettoyage du conduit de l'oreille externe comprenant un manche, un support vissé à une extrémité de ce dernier ou d'une seule pièce avec lui, une pièce nettoyante (un morceau d'étoffe ou de cuir par exemple) échangeable et une bague de maintien de cette pièce nettoyante sur le support. Ladite pièce nettoyante est rapportée sur le support et maintenue grâce à la bague, celle-ci coopérant avec une embase conique du support.

FR-1,582,734 est un dispositif applicateur d'un genre différent dans lequel la tête nettoyante est en matière spongieuse.

EP-A-0,234,061 divulgue un ustensile pour le nettoyage du conduit auditif de l'oreille externe, comprenant une tête nettoyante en caoutchouc enfichée sur la partie terminale d'un bâtonnet dont elle peut être désolidarisée. Cette tête présente, vue de face, une forme de poire et, vue en section, une forme étoilée.

EP-A-0,184,237 décrit un produit du même type, en deux parties également, soit le bâtonnet et une tête nettoyante enfichable, cette dernière pouvant présenter, en outre, une embase remplissant la fonction d'arrêt afin de limiter la pénétration de ladite tête dans le conduit auditif et ainsi éviter de toucher le tympan. Cette tête présente, vue de face, une forme cylindrique à bout arrondi et, vue en section et selon une variante, une forme étoilée également.

DE-OS-41,17,526 va dans le même sens que les deux demandes de brevet européen qui viennent d'être citées. La tête présente de préférence une forme ovale et peut être protégée par une bague coulissante lorsque le produit n'est pas utilisé.

US-1,980,826 décrit un ustensile de nettoyage du conduit auditif externe, comprenant un manche, une garde et une tête nettoyante, interchangeable selon certaines formes d'exécution, cette tête comportant une tige filetée vissée à une extrémité du manche, de sorte à être ajustable quant à la longueur, tout en restant suffisamment liée en rotation lors de l'utilisation. La partie apparente de la tête nettoyante est en caoutchouc, en éponge de caoutchouc ou en feutrine.

La matière préconisée des têtes nettoyantes décrites dans la plupart des antériorités est une matière souple et compacte, le caoutchouc, l'étoffe, le cuir ou une matière équivalente. Mais FR-1,582,734 et US-1,980,826 enseignent l'utilisation d'une matière spongieuse. Dans DE-OS-41,17,526, les nervures ou lamelles de la tête peuvent en outre être pourvues de noppes.

Ces antériorités constituent certes des améliorations par rapport au coton-tige, si l'on considère l'usage spécifique d'hygiène corporelle, mais ils n'écartent pas de manière satisfaisante les inconvénients rencontrés avec les cotons-tiges commentés en premier lieu. En effet, si l'on peut imaginer que les lamelles de caoutchouc se plient en phase d'utilisation - à condition encore que certaines conditions quant aux dimensions desdites lamelles soient respectées (ce qui peut soulever des problèmes de faisabilité et de fiabilité du produit, non évoqués et moins encore résolus dans l'art antérieur) - une adaptation ou conformation continuelle et uniforme de la tête nettoyante à la surface ou au conduit à nettoyer n'est pas donnée. De plus, les têtes nettoyantes ont une forme prédéterminée, fixée et imposée une fois pour toute. Le façonnage spécifique de celles-ci, alors même que leur action, comme on vient de le voir, ne résout pas véritablement les problèmes, et les moyens relativement complexes mis en oeuvre, en particulier dans US-1,980,826, interviennent sensiblement dans les coûts de fabrication de ces instruments, pour les augmenter. Quant à US-1,693,581, l'inconvénient qu'il présente apparaît d'évidence. En effet, on conçoit aisément que lorsque l'instrument tel que décrit et défini est mis en oeuvre, la pièce nettoyante en matière souple et compacte va nécessairement se vriller et glisser autour de son support, lors de l'actionnement en rotation de l'instrument, le seul moyen de rétention ou de retenue étant la bague de maintien. Enfin, les applications restent pratiquement limitées à l'hygiène corporelle, plus particulièrement au nettoyage de conduits auditifs externes.

La présente invention s'est fixée pour objectif de pallier les inconvénients aussi bien techniques qu'économiques dont les ustensiles connus continuent d'être affectés.

Ce but est atteint grâce aux moyens définis dans la revendication indépendante 1.

Les revendications dépendantes définissent des formes particulières et préférées de l'invention, permettant une exploitation optimale de celle-ci.

Une forme d'exécution de l'invention va maintenant être décrite en détail, à titre d'exemple non limitatif, à l'appui du dessin annexé dans lequel
- la Fig. 1: représente une forme possible de l'instrument selon l'invention,
- la Fig. 2 A: montre une forme de l'élément nettoyant, avant montage,
- la Fig. 2 B: montre une autre forme de l'élément nettoyant avant montage,
- les Fig. 3 A et 3 B: représentent une pièce de maintien de l'élément nettoyant,
- la Fig. 4: montre les parties constitutives de l'instrument avant assemblage et
- la Fig. 5: représente l'instrument selon l'invention à l'état assemblé
(remarque étant faite que les pièces et éléments représentés ne sont pas toujours à la même échelle).

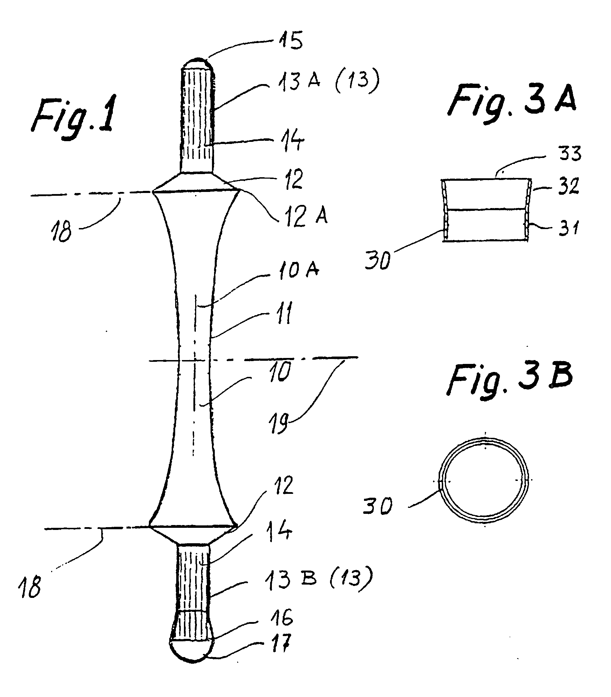

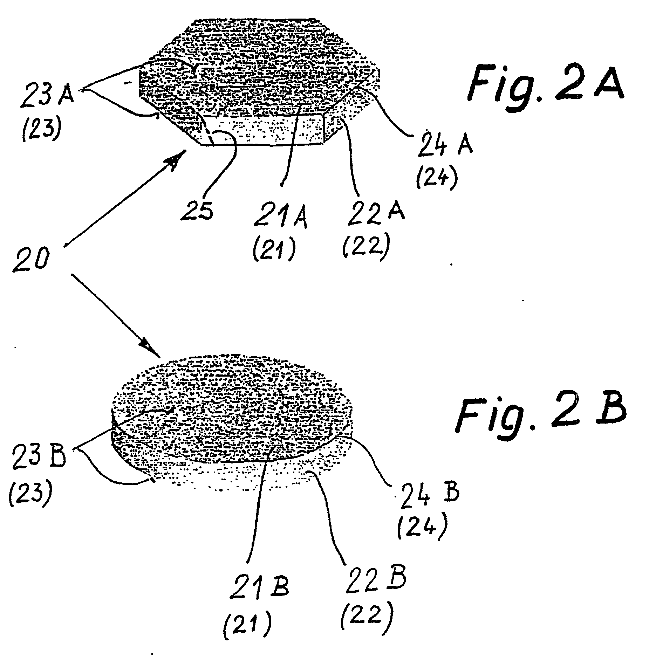

L'ustensile représenté à la figure 1 comprend un manche 10, d'axe de symétrie 10 A et de profil hyperboloïde 11, et au moins à l'une de ses deux extrémités, une embase 12 et un support 13 A, 13 B. Cette embase est tronconique, mais pourrait avoir tout autre profil, notamment hyperboloïde, à l'instar du profil 11 précitée. Les formes des supports ne sont pas nécessairement identiques. Ainsi, selon l'exemple illustré à la figure 1, non limitatif, on voit que le support 13 A est cylindrique, tandis que le support 13 B n'est cylindrique que sur une longueur, la zone terminale comportant un renflement 16 sur l'effet duquel on reviendra. Le bout 15, 17 du support 13 est arrondi. De manière générale, on parlera par la suite de support 13 pour désigner tout support, indifféremment de sa forme. Enfin, l'embase et le support se situent de préférence (mais non nécessairement), dans le prolongement rectiligne de l'axe 10 A.

Le support 13 est destiné à recevoir (dans les conditions exposées plus loin) un élément nettoyant ou traitant 20 dont deux exemples d'exécution sont montrés dans les figures 2 A et 2 B. L'élément 20 est de préférence en matière souple et spongieuse, plus généralement en matière alvéolaire, telle que l'éponge fine. La surface du support 13 (soit l'enveloppe du support, mais non l'extrémité arrondie 15, 17) présente des moyens permettant d'améliorer la prise sur elle de l'élément nettoyant 20. Ces moyens "de prise" ou d'agrippage s'opposent à un libre rotation dudit élément ou du moins à une rotation intempestive. Selon une variante d'exécution, cette surface est pourvue au moins en partie de cannelures longitudinales 14 ou de stries de section triangulaire, connues en soi (et qu'on conviendra d'appeler moyens rapportés), augmentant sensiblement le coefficient de frottement entre les éléments en contact. Selon une autre variante, les moyens de prise, au lieu d'être rapportées, peuvent être inhérentes ou intrinsèques au choix du matériau dans lequel est confectionné le support 13, c'est-à-dire que ces moyens de prise résultent alors d'une propriété dudit matériau, en ce sens que ce dernier a non seulement un coefficient de frottement élevé, mais aussi une dureté et une rigidité suffisantes, eu égard aux contraintes auxquelles peut être soumis ledit support 13. En effet, si, d'une part, l'agrippage doit être assuré, il est aussi nécessaire, d'autre part, que le support 13 soit apte à résister aux forces de compression ou qui tendent à le faire plier lors du montage de l'élément nettoyant 20 sur lui et/ou lors de la mise en oeuvre de l'instrument. Ainsi, le matériau constituant le support 13 pourra être du caoutchouc, une résine, un elastomère, ou encore toute autre matériau apte à être estampé, l'essentiel étant que le matériau choisi allie les conditions requises précitées (quand coefficient de frottement et matière suffisamment rigide). Selon une forme d'exécution de cette seconde variante, seule l'enveloppe du support 13, ou l'enveloppe de la partie terminale du support 13, sera pourvue du matériau choisi précité (à grand coefficient de frottement et rigidité relativement élevée), tandis que le restant du support 13 (donc au moins l'âme ou le noyau) sera en matière rigide quelconque (par exemple en alliage léger). Bien entendu, rien n'empêche que le support 13 présentant des moyens de prise inhérents au matériau choisi comporte également des moyens d'agrippage rapportés du genre de ceux mentionnés plus haut.

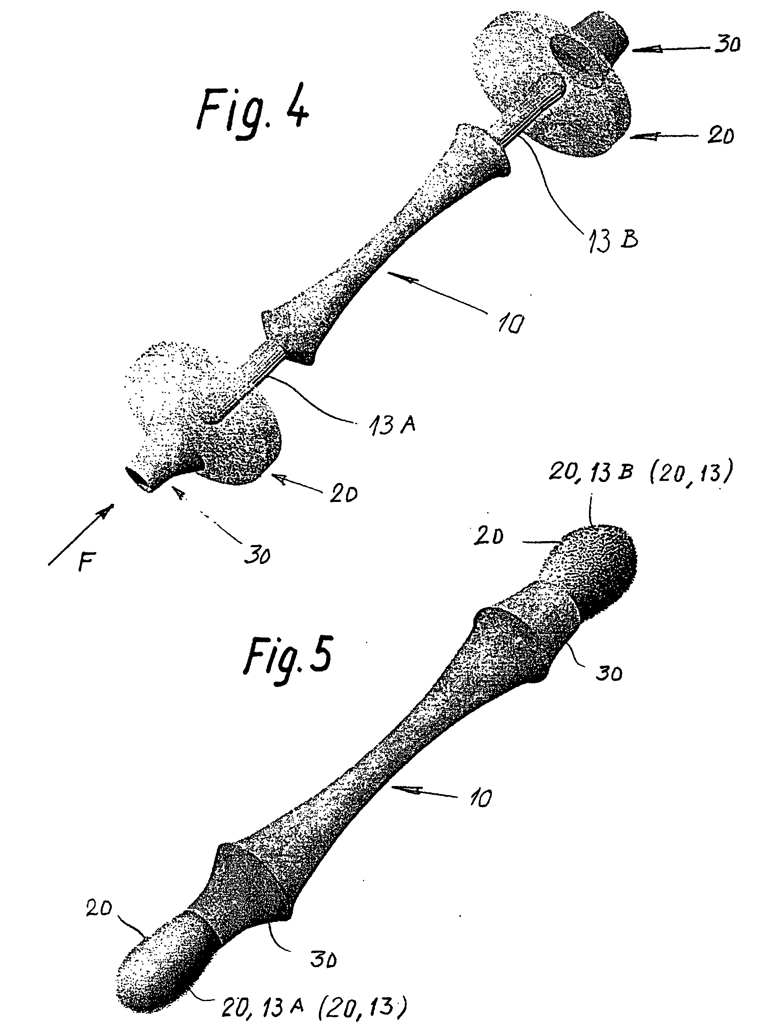

Les figures 2 A et 2 B montrent l'élément nettoyant ou traitant, de référence générale 20, qui a la particularité de se présenter, avant montage sur l'un quelconque des supports 13, sous forme de pièce plate de préférence (ou par exemple légèrement concave). Comme on vient de le dire, cet élément 20 est avantageusement en éponge fine, naturelle ou synthétique (mousse synthétique polyuréthanne de type ester ou éther) travaillant à la compression.L'élement 20 peut se présenter sous diverses formes (contour, dimensions de la surface et de l'épaisseur, sous réserve, quant aux dimensions de la surface, de la précision apportée en fin du présent paragraphe) (voir figures 2A et 2B, formes référencées 21A et 21B respectivement). La figure 2 A montre un élément 20 de pourtour ou contour polygonal, en l'occurrence hexagonal. Ce pourtour porte la référence 22 A, une arête la référence 24 A et les deux surfaces opposées l'une à l'autre la référence 23 A. La figure 2 B montre un élément 20 de pourtour circulaire. Ce pourtour porte la référence 22 B, une arête la référence 24 B et les deux faces opposées l'une à l'autre la reference 23 B. Par la suite, on fera abstraction des lettres affectées à ces références pour désigner de façon générale un élément nettoyant 20, une forme 21, un pourtour 22, une face 23 ou une arête 24. A l'état rabattu ou recourbé sur le support 13 (voir infra), le pourtour 22 de l'élément nettoyant 20 vient s'appliquer contre le support 13 ou la surface d'appui 12, ou se situe au moins dans le voisinage de cette surface, l'élément 20 recouvrant ainsi entièrement ou au moins partiellement ledit support. C'est-à-dire que les formes de l'élément 20 (voir réserve précitée) sont définies de préférence de telle manière qu'à l'état rabattu, ledit pourtour, ou plus précisément une arête 24 (voir aussi la figure 4), ou au moins des portions ce celle-ci, alors resserrée et froncée autour du support 13 ou de l'embase 12, se trouve approximativement à hauteur d'un plan 18 (symbolisé par un trait mixte à la figure 1). Le pourtour 22 peut être taillé en biais (référence 25 à la figure 2 A) . En d'autres termes, les sections droites (perpendiculaires aux surfaces 23), non représentées, de l'élément 20, quelle qu'en soit la forme 21, peuvent être trapézoïdales au lieu d'être rectangulaires. Selon une variante, on peut prevoir que l'élément nettoyant 20 présente, dans la zone centrale, une épaisseur plus importante que sur le restant de la surface, périphérique à cette zone. De cette manière, l'élément nettoyant opposera une meilleure résistance à la force s'exerçant sur lui en cet endroit lors du montage (voir plus loin).

Les figures 3 A et 3 B montrent une coupe et une vue de dessus d'une pièce auxiliaire 30 remplissant une fonction de maintien, à savoir, selon l'exemple, une virole tubulaire destinée à coopérer - lors de la fixation dudit élément sur l'un quelconque des supports 13 - avec, d'une part, l'élément nettoyant 20 et, d'autre part, le manche 10 ou plus précisément le bord 12 A de l'embase 12 sur laquelle la pièce 30 prend appui (voir aussi les figures 4 et 5). Cette pièce 30, qu'on appellera indifférement pièce de maintien ou de retenue, ou virole, peut revêtir diverses formes. Selon la variante décrite, elle présente la forme d'une cloche. C'est dire que la virole 30 se compose d'une partie 31 essentiellement cylindrique et, dans le prolongement de celle-ci, d'une partie 32 essentiellement tronconique dont l'angle au sommet est avantageusement de l'ordre de 60 degrés. A l'instar du support 13, tout ou partie de la surface intérieure de la virole 30 (de préférence uniquement la partie cylindrique 31) comporte avantageusement des moyens permettant d'améliorer la prise sur elle de l'élément nettoyant 20, c'est-à-dire, par exemple, des cannelures longitudinales (non représentées). Selon une exécution préférée, la virole 30 est rigide. Mais on peut également concevoir une pièce de maintien souple, par exemple en matière élastique, exerçant une pression contre l'élément 20 pour retenir ce dernier contre le support 13. Dans ce cas, il va de soi qu'il n'y a pas lieu de la pourvoir de moyens d'agrippage.

Le montage de l'élément nettoyant 20 sur un support 13 s'effectue de manière extrêmement simple et rapide, comme on le comprend à l'appui de la figure 4, grâce, d'une part, aux caractéristiques indiquées plus haut de la matière choisie et, d'autre part, au choix des moyens de retenue préférés de l'élément 20 (le moyen préféré étant, en l'occurrence, une virole rigide). En effet, il suffit, dans un premier temps, de placer l'élément 20 sur la virole 30, de sorte que les axes (non représentés) de ces deux pièces se confondent à peu près (comme on le voit à la figure 4, l'élément 20, et selon une variante la partie la plus épaisse de cet élément, étant posé contre la partie la plus évasée de la cloche, c'est-à-dire contre le rebord 33 (figures 3 A et 4), puis dans un deuxième d'appliquer le milieu de l'ensemble 20, 30 contre l'arrondi 15, 17 du support 13 et enfin, dans un troisième temps, de pousser cet ensemble 20, 30 sur le support 13 en direction de l'embase 12, en exerçant une force dans le sens de la flèche F. Par cette opération, l'élément 20 se plie toujours davantage pour épouser la forme du support 13 et coiffer ce dernier, tandis que la virole est poussée contre l'embase 12 du manche 10. Ici apparaît l'avantage de renforcer l'élément nettoyant dans sa zone centrale, au moyen, par exemple, d'une surépaisseur dudit élément dans ladite zone. Cette manoeuvre effectuée, la virole 30, dont le bord 33 s'appuie contre le bord 12 A de l'embase 12, recouvre donc une partie de l'élément 20, tandis que l'autre partie de cet élément 20 émergeant hors de la partie cylindrique 31 forme, avec le support 13, une tête nettoyante ou traitante 20, 13. Le bord 33 est exécuté de telle sorte qu'il ne détériore pas l'élément 20 lors de ces opérations de montage/démontage. Aussi est-il de préférence arrondi ou pourvue d'un ourlet (ces particularités ne sont pas montrées aux figures 3 A, 3 B, 4). Pour extraire ou désolidariser ledit élément 20 de son support 13, on effectuera l'opération inverse, c'est-à-dire qu'on exercera une force F' en sens inverse de la force F (Fig. 4) et d'intensité équivalente (la force F' n'est pas représentée sur la figure 4), l'élément 20 reprenant automatiquement, au fur et à mesure de sa libération, au moins approximativement, sa forme initiale (plane selon l'exemple d'exécution décrit).

On a vu que le profil du support 13 A diffère de celui du support 13 B. Du fait que l'élément 20 est en matière souple, de préférence en éponge, on aboutit à des têtes nettoyantes de formes différentes (c'est-à-dire, vu dans le sens de l'axe 10 A de la tige 10, de différents diamètres), cela avec un même élément standard 20. Ainsi, l'utilisation pourra bien évidemment agencer indifféremment un jeu de pièces 20, 30 sur l'un des supports, puis placer ce même jeu, par après, sur l'autre support de forme différente, selon le but recherché, une forme pouvant se prêter mieux qu'une autre à une utilisation spécifique, ou encore suite à un nettoyage intensif de l'élément ou des éléments 20. Ces avantages sont d'autant plus intéressants qu'ils se prolongent d'un avantage économique évident, tant en ce qui concerne le coût de fabrication que celui de l'utilisation de l'instrument. Notons dans ce contexte qu'il est bien sûr plus avantageux de pourvoir chacun des supports d'un élément nettoyant 20, l'ustensile se présentant alors comme montré à la figure 5.

L'ensemble 20, 30 reste immobile grâce à l'action d'un groupe de moyen ou, de préférence grâce aux actions conjuguées de ce premier groupe de moyens avec un second groupe de moyens. Le premier groupe de moyens est formé de moyens d'agrippage qui peuvent avantageusement être, selon une variante, les moyens 14 prévus sur le support 13 ou, selon une autre variante, les moyens résultant du matériau même dans lequel le support 13 est (entièrement ou partiellement) fabriqué. En outre, ce premier groupe de moyens peut être complété par des moyens d'agrippage du même genre prévus à l'intérieur de la virole ou prévus par la matière - même de la virole. De manière générale, lorsqu'il s'agit de moyens rapportés, le coefficient de frottement peut encore être augmenté en se contentant d'un façonnage relativement grossier desdits moyens d'agrippage, c'est-à-dire en laissant subsister des aspérités sur la ligne de crête des cannelures). Le second groupe de moyens résulte des forces de réaction de l'élément 20 en éponge sur la virole 30, compte tenu du diamètre d'ouverture du tube 31, ce diamètre devant être adapté aux dimensions du support 13 (c'est-à-dire au diamètre dudit support, si celui-ci est cylindrique) et de l'épaisseur appropriée définie pour l'élément nettoyant 20. Dans la zone de la partie conique 32 de la virole 30, la matière spongieuse peut certes reprendre une certaine expansion, compte tenu du profil de l'embase 12 (présentant, selon l'exemple montré aux fig. 1 et 4, une conicité sensiblement supérieure à celle de la partie 32 de la virole 30), mais cela ne nuit pas à la fiabilité de l'assemblage, puisqu'une poussée reste exercée sur la virole, par la partie de l'élément 20 qui y est enfermée, l'élément 20 ayant tendance à reprendre sa forme plane ou quasi-plane. De plus, la forme en cloche de la virole présente un autre double avantage, celui de l'ergonomie, puisqu'elle permet une manipulation aisée lors du "coiffage" de l'élément 20 sur le support 13 et du "décoiffage", et esthétique, puisqu'elle est en harmonie avec celle du manche 10. On note que la pièce de maintien 30 en assurant la position rabattue de l'élément nettoyant 20 est en même temps retenue elle-même, contre tout déplacement, grâce à ladite poussée exercée sur elle par ledit élément 20.

De manière générale, les dimensions des différentes pièces et parties de pièces 10, 12, 13, 20, 30 composant l'ustensile devront être adaptées les unes aux autres ainsi qu'à l'utilisation ou au domaine d'utilisation envisagé, ce qui est à la portée de l'homme du métier. Quant à la constitution des différentes parties 10, 12, 13 et 30, on peut prévoir tout matériau approprié ou combinaison de matériaux (métal léger, matière synthétique, etc.), sous réserve, bien sûr, du choix du matériau du support 13, selon la variante d'exécution.

Selon une variante d'exécution non représentée, la virole 30 et le manche 10 peuvent comporter des moyens complémentaires de retenue ou d'immobilisation - connus en soi de l'homme du métier (enclipsage, fourches, agrafes, etc.) - coopérant entre eux, pour assurer une liaison avec le manche 10 et, dans le même temps, le maintien des éléments 20 et 30. De tels moyens seront de préférence agencés, d'une part, dans la zone du rebord 33 de la virole et, d'autre part, de l'embase 12 ou la zone 12 A du manche 10. Des moyens de cette nature sont recommandés pour les instruments de grande taille destinés à d'autres applications (voir infra).

Selon une autre forme d'exécution (également non représentée), le nombre de supports 13 peut être augmenté, en choisissant un manche multi-branches. C'est-à-dire, à titre d'exemple, que le manche pourrait présenter la forme d'un croisillon, donc comporter deux branches, l'une d'axe 10, l'autre d'axe 19 (figure 1), chaque branche ayant au moins un support et chaque support pouvant présenter une forme spécifique.

Bien entendu, selon une variante d'exécution, certes moins intéressante, il serait également possible de prévoir une présentation monolithique de l'ustensile, par exemple en collant sur le support 13 l'élément nettoyant 20, avec ou sans virole (auquel cas la fonction du moyen auxiliaire de maintien est remplie par la colle). Lorsque l'usure de l'élément 20 l'exigera, l'utilisateur pourra détacher l'élément usé (ce qui entraînera la destruction au moins partielle de cet élément usé) et le remplacer par un nouvel élément qu'il collera sur le support 13.

Lorsque l'élément 20 est constitué d'une matière spongieuse, il travaille à la compression, de sorte que lors de son application sur la surface à nettoyer ou à traiter ou de son introduction, par exemple dans le conduit auditif externe de l'oreille à nettoyer ou à traiter, il se comprime et au moins une partie de son enveloppe (c'est-à-dire la surface externe et active 23 de la tête 20,13), exerce simultanément sur ladite surface ou ledit conduit une force de réaction perpendiculaire chaque fois au plan tangent à l'enveloppe. En d'autres termes, l'élément 20 est continuellement et uniformément pressé contre la surface ou le conduit lors de l'opération de nettoyage ou de traitement. Aussi le pouvoir nettoyant de la tête 20, 13 est-il optimal, l'éponge se caractérisant par l'alliance, d'une part, d'une certaine dureté propre à la matière elle-même, donc d'un degré abrasif idéal pour l'action de nettoyage et, d'autre part, d'une souplesse procurée par la présence des alvéoles, cette souplesse étant modulée ou fonction de la densité et des dimensions de ces alvéoles, les particules à éliminer (par exemple le cérumen lorsque l'ustensile est utilisé pour nettoyer le conduit auditif externe) étant par ailleurs captées par ces dernières et facilement éliminables par la suite par simple rinçage de la tête 20, 13.

L'autre avantage important de l'ustensile selon l'invention réside dans le fait qu'il se prête de manière idéale aussi bien à une mise en oeuvre "humide" que "sèche", puisque la matière spongieuse permet, soit d'absorber un fluide actif pour une fin déterminée, puis de libérer uniformément ce fluide dans le conduit, la tête nettoyante exerçant simultanément une fonction de répartition par absorption et libération successive et continuelle du fluide actif en plus de la fonction nettoyante, soit exercer une fonction de rinçage ou de séchage.

Un autre avantage est donné par les domaines d'applications multiples que permet l'ustensile selon l'invention, du fait que celui-ci peut être fabriqué à des échelles très différentes, les moyens dans leur principe, leurs formes et leurs fonctions restant les mêmes.

Ainsi, l'ustensile peut être fabriqué dans des dimensions usuelles et connues (ustensile d'une longueur hors tout de l'ordre d'une dizaine de centimètres) à des fins non seulement médicales, vétérinaires, d'hygiène corporelle, de cosmétique et de maquillage, mais aussi à toutes autres fins utilitaires telles que la teinture, la peinture ou le bricolage. D'autres applications, totalement différentes, en particulier dans le domaine du nettoyage ménager ou industriel, ne sont pas moins envisageables. Dans ce cas, il suffira de définir d'autres dimensions pour les éléments constituant l'ustensile, adaptées chaque fois au domaine d'utilisation particulier.

La mise en oeuvre de l'invention dans les domaines d'application les plus divers est d'autant plus intéressante et recommandée que la ou les éléments nettoyants ou têtes nettoyantes peuvent dans tous les cas, après chaque utilisation, être facilement et efficacement rincées, nettoyées voire même, le cas échéant, aseptisées (en écartant la variante, peu intéressante, du collage de l'élément nettoyant sur son support).

L'ustensile selon l'invention allie l'efficacité à une fabrication particulièrement peu coûteuse, alors même que l'on conçoit aisément que sa présentation peut être des plus variées (selon la variante préférée, les différents éléments, manches, éléments nettoyants et viroles peuvent être acquis séparément) et que ses applications possibles sont pratiquement infinies.

## Revendications

1. Ustensile de nettoyage ou de traitement à usages et applications multiples, notamment d'hygiène corporelle, comportant
- au moins une manche (10),
- au moins un support (13) destiné à être coiffé d'un élément nettoyant ou traitant (20) pour former, avec le support (13) une tête nettoyante ou traitante (20, 13), l'élément nettoyant ou traitant (20) étant une pièce interchangeable constituée d'une matière souple lui permettant d'adopter toute forme voulue sous l'effet de forces appliquées sur elle, de sorte que ledit élément (20) peut épouser la forme du support (13) lorsqu'il est agencé sur celui-ci pour le coiffer au moins partiellement et y être retenu par une pièce de maintien (30), **caractérisé en ce que** l'élément nettoyant ou traitant (20) se présente avant son agencement sur le support (13) sous une forme au moins approximativement plate, au pourtour (22) circulaire ou polygonal, que cet élément (20) peut reprendre au moins approximativement sa forme initiale en l'absence desdites forces, ladite matière travaillant à la compression, **en ce que** le support (13) comprend des moyens d'agrippage (14) s'opposant à une libre rotation de l'élément (20) agencé sur lui et **en ce que** la pièce de maintien (30) assurant le maintien de l'élément (20) sur le support (13)est retenue elle-même par l'action exercée sur elle par ledit élément (20).

2. Ustensile selon la revendication 1, **caractérisé en ce que** les moyens (14) du support (13) s'opposant à la rotation de l'élément (20) de la tête (20, 13) sont des moyens rapportés, tels que cannelures ou stries à section triangulaire ayant une action d'agrippage sur ledit élément (20).

3. Ustensile selon la revendication 1, **caractérisé en ce que** les moyens d'agrippage (14) sont des moyens intrinsèques résultant du choix d'un matériau approprié dans lequel est constitué, au moins pour partie, le support (13), lesdits moyens intrinsèques pouvant en outre comporter des moyens rapportés tels que cannelures ou stries.

4. Ustensile selon la revendication 3, **caractérisé en ce que** le matériau présentant les moyens intrinsèques allie une rigidité définie à un coefficient de frottement défini, ce matériau étant de préférence une résine ou un élastomère.

5. Ustensile selon l'une des revendications 1 à 4, **caractérisé en ce que** chacun des supports (13) présente une forme spécifique.

6. Ustensile selon l'une des revendications 1 à 5, **caractérisé en ce que** la matière constituant l'élément (20) est alvéolaire, de préférence de l'éponge fine.

7. Ustensile selon l'une des revendications 1 à 6, **caractérisé en ce que** la pièce de maintien (30) recouvre partiellement l'élément (20) et peut comporter des moyens s'opposant à une libre rotation mutuelle de ladite pièce (30) et dudit élément (20).

8. Ustensile selon l'une des revendications 1 à 7, **caractérisé en ce que** la pièce de maintien (30) présente une forme approximative en cloche, avec une partie essentiellement cylindrique (31) et une partie essentiellement conique (32) dont le bord évasé (33) vient en contact avec le manche (10).

9. Ustensile selon l'une des revendications 1 à 8, **caractérisé en ce que** les dimensions de l'élément (20) sont telles que lorsqu'il est agencé sur le support (13), au moins des portions du pourtour (22) dudit élément se situent approximativement dans un plan (18) du manche (10).

10. Ustensile selon l'une des revendications 1 à 9, **caractérisé en ce que** le manche (10) présente un profil hyperboloïde et que le support (13) est relié audit manche par une embase (12) sur laquelle prend appui l'élément de maintien (30).

11. Ustensile selon la revendication 9 **caractérisé en ce que** le plan (18) se situe dans une zone (12 A) la plus évasée de l'embase (12).

12. Ustensile selon l'une des revendications 1 à 11, **caractérisé en ce que** chaque support (13) se situe de préférence dans le prolongement de l'axe (10 A) du manche (10).

13. Ustensile selon l'une des revendications 5 à 12, **caractérisé en ce que** la pièce de maintien (30) et le manche (10) comportent des moyens complémentaires de retenue coopérant entre eux.

14. Ustensile selon la revendication 13, **caractérisé en ce que** le moyen complémentaire de retenue de l'élément (20) sur le support (13) est la colle.

15. Ustensile selon l'une des revendications 1 à 14, **caractérisé en ce qu'**il comporte un manche à plusieurs branches dont au moins l'une des deux extrémités chaque fois comporte un support (13).

## Patentansprüche

1. Reinigungs- bzw. Behandlungsutensil für vielfältige Anwendungen, namentlich für die Körperpflege, mit
- mindestens einem Schaft (10),
- mindestens einem Träger (13), auf den ein Reinigungs- bzw. Behandlungselement (20) aufgesetzt wird, um mit dem Träger (13) einen Reinigungs- bzw. Behandlungskopf (20, 13) zu bilden, wobei das Reinigungs- bzw. Behandlungselement (20) ein auswechselbares Teil aus einem weichen Material ist, wodurch sich das Teil unter der Einwirkung von darauf ausgeübten Kräften an jede gewünschte Form anpassen kann, so dass das genannte Element (20) die Form des Trägers (13) annehmen kann, wenn es auf denselben aufgesetzt ist und ihn zumindest teilweise bedeckt, während es durch eine Halterung (30) befestigt ist, **dadurch gekennzeichnet, dass** das Reinigungs- bzw. Behandlungselement (20) vor dessen Anbringung am Träger (13) in zumindest annähernd flacher Form vorliegt und runde oder polygonale Umrisse (22) aufweist, dass das Element (20) ohne die genannten Kräfte zumindest annähernd zu seiner ursprünglichen Form zurückkehrt, wobei das genannte Material auf Druck beansprucht wird, dass der Träger (13) Haftmittel (14) aufweist, welche eine freie Drehung des daran angebrachten Elements (20) verhindern, und dass die Halterung (30), welche die Befestigung des Elements (20) am Träger (13) bewirkt, ihrerseits durch die durch genanntes Element (20) auf sie ausgeübte Wirkung festgehalten ist.

2. Utensil nach Anspruch 1, **dadurch gekennzeichnet, dass** die Mittel (14) des Trägers (13) zur Verhinderung einer Drehung des Elements (20) des Kopfs (20, 13) angebrachte Mittel sind, beispielsweise Rillen dreieckigen Querschnitts, welche eine Haftwirkung auf das genannte Element (20) ausüben.

3. Utensil nach Anspruch 1, **dadurch gekennzeichnet, dass** die Haftmittel (14) immanente Mittel sind, welche aus der Wahl des geeigneten Materials folgen, aus dem der Träger zumindest teilweise besteht, wobei diese immanenten Mittel zusätzlich angebrachte Mittel wie beispielsweise Rillen aufweisen können.

4. Utensil nach Anspruch 3, **dadurch gekennzeichnet, dass** das Material mit den immanenten Mitteln eine bestimmte Steifigkeit und einen bestimmten Reibungswert vereinigt, wobei es sich vorzugsweise um ein Harz oder ein Elastomer handelt.

5. Utensil nach einem der Ansprüche 1 bis 4, **dadurch gekennzeichnet, dass** jeder Träger (13) jeweils eine spezifische Form aufweist.

6. Utensil nach einem der Ansprüche 1 bis 5, **dadurch gekennzeichnet, dass** das Element (20) aus Schaumstoff besteht, bevorzugt aus Feinschaumstoff.

7. Utensil nach einem der Ansprüche 1 bis 6, **dadurch gekennzeichnet, dass** die Halterung (30) das Element (20) teilweise überdeckt und Mittel zur Verhinderung einer freien Drehung der Halterung (30) gegenüber dem Element (20) aufweisen kann.

8. Utensil nach einem der Ansprüche 1 bis 7, **dadurch gekennzeichnet, dass** die Halterung (30) annähernd glockenförmig ausgebildet ist und einen im wesentlichen zylindrischen Teil (31) und einen im wesentlichen konischen Teil (32) aufweist, dessen erweiterter Rand (33) mit dem Schaft in Berührung gelangt.

9. Utensil nach einem der Ansprüche 1 bis 8, **dadurch gekennzeichnet, dass** die Abmessungen des Elements (20) derart gewählt sind, dass mindestens Teile des Rands (22) des Elements ungefähr in einer Ebene (18) des Schafts (10) liegen, wenn dieses am Träger (13) angebracht ist.

10. Utensil nach einem der Ansprüche 1 bis 9, **dadurch gekennzeichnet**, das der Schaft (10) ein hyperboloides Profil aufweist und der Träger (13) mit dem Schaft über einen Absatz (12) verbunden ist, auf dem die Halterung (30) aufliegt.

11. Utensil nach Anspruch 9, **dadurch gekennzeichnet, dass** die Ebene (18) im breitesten Bereich (12 A) des Absatzes (12) liegt.

12. Utensil nach einem der Ansprüche 1 bis 11, **dadurch gekennzeichnet, dass** die Träger (13) vorzugsweise jeweils in der Verlängerung der Achse (10 A) des Schafts (10) angeordnet sind.

13. Utensil nach einem der Ansprüche 5 bis 12, **dadurch gekennzeichnet, dass** die Halterung (30) und der Schaft (10) komplementäre Haltemittel aufweisen, welche miteinander zusammenwirken.

14. Utensil nach Anspruch 13, **dadurch gekennzeichnet, dass** das komplementäre Haltemittel des Elements (20) am Träger (13) Klebstoff ist.

15. Utensil nach einem der Ansprüche 1 bis 14, **dadurch gekennzeichnet, dass** es einen Schaft mit mehreren Armen aufweist, wobei mindestens eines der zwei Enden jedes Arms einen Träger besitzt.

## Claims

1. Cleaning or treating utensil for multiple uses and applications, particularly for personal hygiene, comprising
- at least one stem (10),
- at least one support (13) destined to be covered with a cleaning or treating element (20) in order to form a cleaning head (20, 13) with the support (13), the cleaning or treating element (20) being an interchangeable piece constituted by a supple material allowing its adaptation to any desired shape under the effect of forces applied thereto, such that the element (20) is capable of taking the shape of the support (13) when it is disposed on the latter while covering it at least partially and being retained thereon by a retaining member (30), **characterised in that** prior to its attachment to the support (13), the cleaning or treating element (20) is at least approximately in a flat form with a circular or polygonal circumference (22), **in that** the element (20) is capable of returning at least approximately to its initial shape in the absence of the mentioned forces, the mentioned material being under pressure, **in that** the support (13) comprises gripping means (14) preventing a free rotation of the element (20) arranged thereon, and **in that** the retaining member (30) providing the retention of the element (20) on the support (13) is retained itself by the action exerted thereon by the element (20).

2. Utensil according to claim 1, **characterised in that** the means (14) of the support (13) for preventing the rotation of the element (20) of the head (20, 13) are additional means such as grooves or serrations having a triangular section and providing a gripping action on the element (20).

3. Utensil according to claim 1, **characterised in that** the gripping means (14) are intrinsic means resulting from the choice of an appropriate material of which the support (13) is composed at least partially, these intrinsic means being possibly provided furthermore with additional means such as grooves or serrations.

4. Utensil according to claim 3, **characterised in that** the material comprising the intrinsic means combines a defined rigidity and a defined friction coefficient, the material preferably being a resin or an elastomer.

5. Utensil according to one of claims 1 to 4, **characterised in that** each one of the supports (13) has a specific shape.

6. Utensil according to one of claims 1 to 5, **characterised in that** the material constituting the element (20) is a cellular material, preferably fine sponge.

7. Utensil according to one of claims 1 to 6, **characterised in that** the retaining member (30) partially covers the element (20) and may comprise means for preventing a free mutual rotation of the piece (30) and of the element (20).

8. Utensil according to one of claims 1 to 7, **characterised in that** the retaining member (30) is approximately bell-shaped, comprising an essentially cylindrical portion (31) and an essentially conical portion (32) whose widened rim (33) comes into contact with the stem (10).

9. Utensil according to one of claims 1 to 8, **characterised in that** the dimensions of the element (20) are such that when it is arranged on the support (13), at least parts of the circumference (22) of the element are situated in a plane (18) of the stem (10) approximately.

10. Utensil according to one of claims 1 to 9, **characterised in that** the stem has a hyperboloid profile, and **in that** the support (13) is connected to the stem by a shoulder (12) on which the holding element (30) is resting.

11. Utensil according to claim 9, **characterised in that** the plane (18) is situated within the widest area (12 A) of the shoulder (12).

12. Utensil according to one of claims 1 to 11, **characterised in that** each support (13) is preferably situated in the prolongation of the axis (10 A) of the stem (10) .

13. Utensil according to one of claims 5 to 12, **characterised in that** the retaining member (30) and the stem (10) comprise complementary retaining means cooperating with each other.

14. Utensil according to claim 13, **characterised in that** the complementary retaining means of the element (20) on the support (13) is an adhesive.

15. Utensil according to one of claims 1 to 14, **characterised in that** it comprises a stem having several branches, at least one of the two ends of each branch being provided with a support (13).
